# EUROPÄISCHE PATENTSCHRIFT

(11) **EP 2 531 113 B1**
(45) Veröffentlichungstag und Bekanntmachung des Hinweises auf die Patenterteilung: **03.12.2014**
(21) Anmeldenummer: 12700369.7
(22) Anmeldetag: 12.01.2012
(51) Int. Cl.: A61B 17/00, A61B 17/12

(54) **VERSCHLUSS VON UNGEWOLLTEN ÖFFNUNGEN IM HERZEN**
OCCLUSION DEVICE FOR UNDESIRED OPENINGS IN THE HEART
FERMETURE POUR OUVERTURES INDÉSIRABLES DANS LE C UR

(30) Priorität: 12.01.2011 DE 202011001366 U
(43) Veröffentlichungstag der Anmeldung: 12.12.2012
(73) Patentinhaber: Osypka, Peter, 79618 Rheinfelden-Herten (DE)
(72) Erfinder: Osypka, Peter, 79618 Rheinfelden-Herten (DE)
(74) Vertreter: Kunst, Manuel Nikolaus Johannes
(86) Internationale Anmeldenummer: PCT/EP2012/000120
(87) Internationale Veröffentlichungsnummer: WO 2012/095314

(56) Entgegenhaltungen:
- EP-A1- 1 982 655
- EP-A1- 2 399 524
- DE-A1-102006 050 385
- DE-A1-102008 022 673
- US-A1- 2009 187 214

## Beschreibung

Die Erfindung betrifft einen Verschluss, für ungewollte Öffnungen im Herzen (ASD, PFO, VSD) mit zwei gegeneinander beabstandeten flexiblen verschweißten, als Verschlussscheiben dienenden Schirmen aus einem Geflecht von Metalldrähten, womit im menschlichen Herzen Öffnungen verschließbar und die bei Bedarf und Fehlplatzierung zu bergen und wieder neu zu platzieren sind, wobei die Verbindung zwischen den Schirmen oder Verschlussscheiben frei von Klammern, Fassungen oder Gewindehülsen ist und durch die Verschweißung unmittelbar gebildet ist, und die Metalldrähte der die Schirme oder Verschlussscheiben bildenden Geflechte auf den einander zugewandten Seiten der Schirme oder Verschlussscheiben miteinander verschweißt sind.

Verschlüsse für ungewollte Öffnungen mit Vorrichtungen aus Metall im menschlichen Körper sind durch US 5 846 261, DE 695 34 505 oder US 5 725 552 bekannt. Bei diesen vorbekannten Verschlüssen sind entweder eine oder zwei Klemmen für die Drähte und Litzen des Metallgeflechts vorhanden, die in die Blutbahn hineinragen und für die Fixierung an einem Katheder ist ein Gewinde erforderlich, was vor allem ein eventuelles Bergen nach einer Fehlplatzierung schwierig macht.

Diese letzteren Nachteil weist auch das Verschlusselement gemäß DE 2008 022 673 A1 auf. Zwar sind dabei die Klemmen oder Fassungen für die Fäden oder Drähte des Geflechts einander zugewandt, müssen aber miteinander verschraubt oder verschweißt werden, was Klemmen oder Fassungen für die Fäden oder Drähte erforderlich macht und bei der gegenseitigen Verschraubung oder Verschweißung einen zusätzlichen Aufwand bedeutet.

Aus der älteren, nicht vorveröffentlichten EP 2 399 524 A1, die ein Dokument gemäß Artikel 54(3) EPÜ darstellt, ist ein vergleichbarer Verschluss für ungewollte Öffnungen im Herzen bekannt, wobei für die Verbindung der beiden Verschlussscheiben oder Schirme ein zwischenliegendes Befestigungsglied vorgesehen ist, an welchem die Drähte auf unterschiedliche Weise befestigt sein können. Dabei kann das Befestigungsglied auch eine Schweißung sein. Es muss also dieses zwischenliegende Befestigungsglied vorgesehen und mit den Drähten verbunden werden, was ebenfalls einen zusätzlichen Aufwand bedeutet. Um den Verschluss Depositionieren zu können, muss dabei an einem mit einer Verschlussscheibe verbundenen Verbindungsglied angegriffen werden, was zu Verformungen und auch zu einer Veränderung der Verschlussscheibe führen kann.

Es besteht daher die Aufgabe, einen Verschluss für ungewollte Öffnungen im Herzen mit zwei im Wesentlichen parallelen Verschlussscheiben oder Schirmen zu schaffen, womit ein zuverlässiges Verschließen eines ASDs oder PFOs oder VSBs des Herzens ermöglicht wird, ohne dass dabei eine Metallklemme, Fassung oder Gewindehülse in die Blutbahn hineinragt.

Vor allem soll ermöglicht werden, den Verschluss auf einfache Weise repositionieren zu können.

Diese Aufgabe wird mit den Mitteln und Merkmalen des Patentanspruchs 1 gelöst. Für eine einfache Repositionierung ist dabei der eingangs definierte Verschluss dadurch gekennzeichnet, dass an dem in Gebrauchsstellung rechten Schirm oder der rechts angeordneten Verschlussscheibe ein nach Außen führender Faden oder Doppelfaden vorgesehen ist. Zum Bergen des Verschlusses bei einer Fehlplatzierung genügt es also, an diesem Faden oder Doppelfaden eine entsprechende Zugkraft auszuüben, um den Verschluss wieder nach Außen zu transportieren. Danach kann er in bekannter üblicher Weise wieder positioniert werden, wobei der Benutzer dabei dann auf eine verbesserte Positionierung achten kann.

Demgemäß ergibt sich auch ein Verschluss mit zwei Verschlussscheiben, die flexibel miteinander verbunden sind, weil zwischen Ihnen keine Klemmen oder Klammern oder Fassungen vorgesehen sind, die verschraubt werden müssten, was außerdem zu einem verringerten Gewicht führt. Auf Grund der hohen Flexibilität und des geringen Gewichts kann ein derartiger Verschluss in der vorbeschriebenen Weise auch aus einer eventuellen Fehlplatzierung wieder zurückgezogen und also geborgen und neu platziert werden.

Dennoch ergibt sich ein zuverlässiger Verschluss einer ungewollten Öffnung im Herzen, der gegebenenfalls auch bei anderen Öffnungen im Körper eines Menschen anwendbar sein kann, ohne dass zum Beispiel in die Blutbahn der Vorhöfe eines solchen Herzens mit einem solchen Verschluss Metallklammern, Fassungen oder Gewindehülse hineinragen, wobei dennoch eine eventuelle Repositionierung einfach durchgeführt werden kann.

Günstig ist es dabei, wenn der Faden oder Doppelfaden mehrere Drähte des Geflechts umgreift oder umfasst. Entsprechend gut kann die Zugkraft des Fadens oder Doppelfadens auf das Geflecht und damit auf den Verschluss ausgeübt werden, ohne dass die Struktur des Verschlusses oder seines Geflechts nachteilig verändert wird.

Eine zweckmäßige Ausführungsform der Erfindung kann dabei darin bestehen, dass die Metalldrähte der die Schirme oder Verschlussscheiben bildenden Geflechte auf den einander zugewandten Seiten der Schirme oder Verschlussscheiben miteinander verschweißt sind. Die Schweißstelle befindet sich dann also in dem Bereich mit der verminderten radialen Abmessung zwischen den beiden Verschlussscheiben, welcher Bereich in Gebrauchsstellung in der zu verschließenden Öffnung angeordnet wird, wie es auch aus DE 10 2008 022 673 A1 bekannt ist, wo allerdings in diesem Bereich mit verminderter radialer Abmessung die beiden zu verschraubenden Fassungen der beiden Verschlussscheiben angeordnet sind.

Das Material der verschweißten Schirme oder Verschlussscheiben und das diese bildende Geflecht kann aus Nitinoldraht bestehen, der sich in der Medizintechnik bewährt hat.

In der Regel sind die beiden beabstandeten Schirme oder Verschlussscheiben - wie aus DE 10 2008 022 673 A1 bekannt - etwa gleich groß und von übereinstimmender Form, in der Regel etwa diskusscheibenartig. Eine abgewandelte Anordnung kann jedoch vorsehen, dass die beiden beabstandeten Schirme oder Verschlusscheiben unterschiedliche Größen und/oder Formen haben. Somit lassen sie sich insbesondere bei ihrer Herstellung an entsprechende anatomische Gegebenheiten anpassen.

Eine zweckmäßige Ausführungsform der Erfindung kann vorsehen, dass an jedem Schirm oder jeder Verschlussscheibe mehrere Drähte der Geflechte miteinander verdrillt und verdrillte Drähte der einen Verschlussscheibe mit solchen der anderen Verschlussscheibe verschweißt sind. Da die Drähte solcher Verschlüsse und Verschlussscheiben dünn gewählt werden, ist ein Verschweißen dieser Drähte besonders gut möglich, wenn die Schweißstelle durch verdrillte Drähte einen etwas vergrößerten Querschnitt gegenüber dem eines Einzeldrahtes haben.

Wie bereits erwähnt, ist die Verbindung zwischen den Schirmen oder Verschlussscheiben zweckmäßigerweise und in vorteilhafter Weise frei von Klammern, Fassungen oder Gewindehülsen und wird durch die Verschweißung unmittelbar gebildet. Diese Verbindung der beiden Scheiben ist einerseits von hoher Festigkeit, andererseits aber flexibel und kann sich somit gut an die Anatomie im Bereich der zu verschließenden Öffnung anpassen lassen.

Eine weitere Ausgestaltung der Erfindung kann vorsehen, dass die Schweißverbindung oder die Stelle der Verschweißung der verdrillten Drähte mit aushärtendem Klebstoff und/oder flexiblem Kunststoff ummantelt ist. Am einfachsten ist es dabei, wenn die gesamte Verbindung zwischen den beiden Verschlussscheiben eine solche Ummantelung aufweist, die durch ihre Verbindung mit den von ihr umfassten Drähten und Schweißstellen deren Zugefestigkeit, Dauerfestigkeit und Reißfestigkeit erhöht.

Eine weitere Ausgestaltung der Erfindung insbesondere zur Verbesserung der Abdichtung kann darin bestehen, dass in den Schirm oder die Verschlussscheibe für wenigstens einen der Vorhöfe eine Kunststoffscheibe integriert ist. Beispielsweise kann eine solche zusätzlich dichtende Kunststoffscheibe in die Verschlussscheibe für den rechten Vorhof in einem Herzen integriert sein, wo der Blutdruck geringer als im linken Vorhof ist. Es kann aber auch Fälle geben, in denen eine solche zusätzlich dichtende Kunststoffscheibe in die Verschlussscheibe für den linken Vorhof integriert wird und schließlich könnten auch in beiden Verschlussscheiben Kunststoffscheiben vorgesehen sein.

Die Verschlussscheibe oder der Schirm, der die Kunststoffscheibe in Gebrauchsstellung enthält, kann für deren Einsetzen bereichsweise offen sein. Es kann also in dem Geflecht eine gewisse Einsetzöffnung von den Drähten freigehalten bleiben, um durch eine so gebildete Öffnung die Kunststoffscheibe einführen zu können.

Nachstehend sind Ausführungsbeispiele der Erfindung anhand der Zeichnung näher beschrieben. Es zeigt in schematisierter Darstellung:

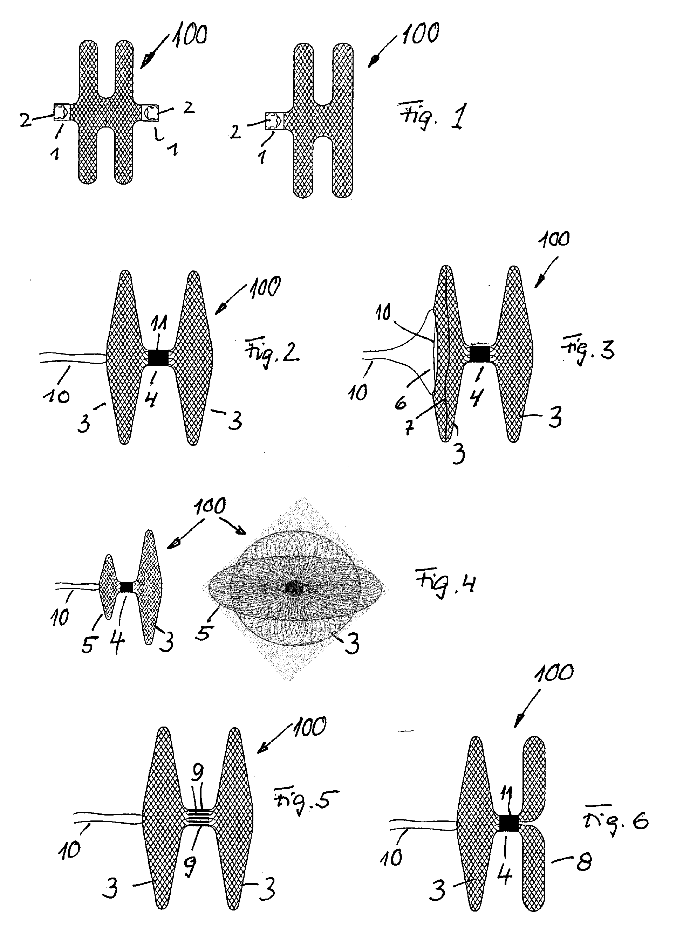

- Fig. 1: zwei beispielhafte Ausführungsformen bekannter Verschlüsse für ungewollte Öffnungen im Herzen mit jeweils zwei beabstandeten, etwa parallelen Schirmen oder Verschlussscheiben aus einem Metallgeflecht, wobei außenseitig zumindest eine beziehungsweise an beiden Seiten eine Klemme oder Klammer zum Fassen der Drähte des Geflechts vorgesehen sind,
- Fig. 2: eine Ausführung eines erfindungsgemäßen Verschlusses für ungewollte Öffnungen im Herzen mit zwei beabstandeten, jeweils aus einem Geflecht gebildeten Verschlussscheiben, deren Geflechte ohne Klammern, Fassungen oder Gewindehülsen an den einander zugewandten Seiten miteinander verschweißt sind,
- Fig. 3: eine abgewandelte Ausführungsform des erfindungsgemäßen Verschlusses mit zwei beabstandeten, jeweils aus einem Geflecht gebildeten Verschlussscheiben, wobei die Verschlussscheibe für den rechten Vorhof teilweise offen ist und eine durch diese Öffnung eingeführte Kunststoffscheibe enthält,
- Fig. 4: eine abgewandelte Ausführungsform des erfindungsgemäßen Verschlusses mit zwei beabstandeten, aus Geflecht gebildeten Verschlussscheiben, die unterschiedliche Formen und/oder Größen haben, in Seitenansicht mit Blick auf die Schmalseiten der beiden parallelen Verschlussscheiben und in Draufsicht auf eine Verschlussscheibe und die aus diesem Blickwinkel dahinter befindliche zweite Verschlussscheibe,
- Fig. 5: eine Ausführungsform des erfindungsgemäßen Verschlusses mit zwei beabstandeten, aus Geflecht gebildeten Verschlussscheiben, die im Bereich ihrer gegenseitigen Verbindung miteinander mehrere Schweißverbindungen haben, sowie
- Fig. 6: eine Ausführungsform des erfindungsgemäßen Verschlusselements mit zwei Verschlussscheiben, wobei für den linken Vorhof eine geänderte oder abgewandelte Scheibenform vorgesehen ist.

Bei der nachfolgenden Beschreibung der unterschiedlichen Ausführungsformen erhalten hinsichtlich ihrer Funktion übereinstimmende Teile auch bei abgewandelter Gestaltung übereinstimmende Bezugszahlen.

Ein im Ganzen mit 100 bezeichneter Verschluss von ungewollten Öffnungen in einem menschlichen Herzen (ASD, PFO, VSD) weist zwei zueinander beabstandete, flexible, als Verschlussscheiben 3 dienende Schirme aus einem Geflecht von Metalldrähten 2 auf, womit im menschlichen Herzen Öffnungen etwa in der Weise verschließbar sind, wie es aus DE 10 2008 022 673 A1 und auch dem darin genannten Stand der Technik bekannt ist.

Die Verschlüsse 100 gemäß Fig. 1 weisen Klemmen 1 und Gewinde 2 auf, die im implantierten Zustand in die Vorhöfe des Herzens hineinragen.

Die Fig. 2 bis 6 mit erfindungsgemäßen Verschlüssen 100 sind demgegenüber so gestaltet, dass solche Klemmen 1 und Gewinde 2 vermieden werden.

Fig. 2 zeigt dabei eine Ausführungsform des Verschlusses 100 mit zwei etwa parallelen, beabstandeten, aus einem Geflecht gebildeten Verschlussscheiben 3, deren Geflechte und Drähte an der Stelle der geringsten radialen Abmessung zwischen den beiden Verschlussscheiben 3 miteinander verschweißt sind. Diese Verschweißung der Drähte der Geflechte ist mit der Bezugszahl 4 gekennzeichnet.

Dabei erkennt man in Fig. 2 wie auch in den Fig. 3 bis 6 außerdem einen an der in Gebrauchsstellung rechten Verschlussscheibe 3 angreifenden Doppelfaden 10, der zum Bergen oder Repositionieren des Verschlusses 100 dient. An diesem Doppelfaden 10 kann der Benutzer eine Zugkraft auf den Verschluss 100 ausüben und ihn gegebenenfalls sogar wieder ganz entfernen und in bekannter Weise erneut einsetzen, falls zunächst eine Fehlplatzierung vorgelegen hat.

Dabei ist angedeutet, dass dieser Doppelfaden 10 mehrere Drähte des Geflechts umfassen kann, um die aufzubringende Zugkraft gut und ohne Strukturveränderungen des Verschlusses 10 einleiten zu können.

Fig. 3 zeigt einen Verschluss 100, bei welchem die Verschlussscheibe 3 für den rechten Vorhof in ihrem Geflecht eine Öffnung 6 hat. Da der Blutdruck im rechten Vorhof stets geringer als im linken Vorhof ist, kann dort der Schirmaufbau, also der Aufbau des Schirmes oder der Verschlussscheibe 3, so gestaltet werden, dass man zur Abdichtung eine dünne Kunststoffscheibe 7 integrieren kann, wie es in Fig. 3 angedeutet ist. Diese Kunststoffscheibe 7 kann durch die Öffnung 6 eingeführt werden. Aufgrund der Öffnung 6 hat der Doppelfaden 10 einen verbreiterten Angriff an dieser Verschlussscheibe 3 im Randbereich der Öffnung 6.

Fig. 4 zeigt eine abgewandelte Ausführungsform des Verschlusses 100 in zwei verschiedenen Ansichten. Die linke Ansicht entspricht den Ansichten gemäß den übrigen Figuren, wobei deutlich wird, dass eine hinsichtlich ihrer Form gegenüber der Form der Verschlussscheibe 3 unterschiedliche Verschlussscheibe 5 vorgesehen sein kann. Betrachtet man nur den linken Teil der Fig. 4, kann es sich um eine insgesamt kleinere Verschlussscheibe 5 beispielsweise wiederum mit etwa kreisrunder Form wie bei den übrigen Verschlussscheiben 3 handeln, jedoch zeigt Fig. 4 rechts, dass in diesem Falle die Verschlussscheibe 5 eine etwas elliptische Form hat und sich dadurch von der Kreisform der Verschlussscheibe 3 unterscheidet. Dadurch können unterschiedliche anatomische Verhältnisse berücksichtigt werden.

Fig. 5 zeigt eine zweckmäßige Ausführungsform des Verschlusses 100, wobei die Drähte des Geflechts der beiden Verschlussscheiben 3 im Bereich zwischen diesen beiden Verschlussscheiben 3 durch mehrere Schweißungen 9 verbunden sind. Diese mehreren Schweißungen 9 sind durch parallele Linien in diesem Verbindungsbereich zwischen den beiden Verschlussscheiben 3 angedeutet.

Fig. 6 zeigt eine Ausführungsform des Verschlusses 100, wobei eine der beiden beabstandeten Verschlussscheiben 3 eine geänderte Scheibenform hat und diese geänderte Verschlussscheibe 8 für den linken Vorhof bestimmt ist. Dabei ist wiederum die Verschweißung 4 gemäß den Fig. 2 bis 4 vorgesehen.

Die Schweißverbindung 4 beziehungsweise die mehreren Schweißungen 9 können dabei mit aushärtendem Klebstoff und/oder flexiblem Kunststoff ummantelt sein. Die in den Zeichnungen jeweils dargestellte Schweißstelle 4 kann dabei so verstanden werden, dass ihr schwarzer Bereich eine Ummantelung 11 der Schweißstelle 4 oder gegebenenfalls auch von Einzelschweißungen 9 bedeutet.

Der Verschluss 100 für ungewollte Öffnungen im Herzen (ASD, PFO, VSD) hat zwei beabstandete, flexible Verschlussscheiben 3, die aus einem Geflecht von Metalldrähten gebildet sind. Damit können vor allem im menschlichen Herzen zum Beispiel zwischen zwei Vorhöfen bestehende Öffnungen verschließbar sein, wobei der Verschluss 100 bei Bedarf oder Fehlplatzierung auch wieder geborgen und neu platziert werden kann. Die Verschlussscheiben sind dabei an den einander zugewandten Stellen im Bereich der geringsten radialen Ausdehnung des Verschlusses 100 miteinander durch Verschweißung verbunden, so dass Klemmen, Klammern und/oder Gewinde vermieden werden.

## Patentansprüche

1. Verschluss (100) für ungewollte Öffnungen im Herzen (ASD, PFO, VSD) mit zwei gegeneinander beabstandeten flexiblen verschweißten als Verschlussscheiben (3) dienenden Schirmen aus einem Geflecht von Metalldrähten, womit im menschlichen Herzen Öffnungen verschließbar und die bei Bedarf und Fehlplatzierung zu bergen und wieder neu zu platzieren sind, wobei die Verbindung zwischen den Schirmen oder Verschlussscheiben (3) frei von Klammern, Fassungen oder Gewindehülsen ist und durch eine Verschweißung (4) unmittelbar gebildet ist, und die Metalldrähte der die Schirme oder Verschlussscheiben (3) bildenden Geflechte auf den einander zugewandten Seiten der Schirme oder Verschlussscheiben (3) miteinander verschweißt sind, wobei an dem in Gebrauchsstellung rechten Schirm oder der rechts angeordneten Verschlussscheibe (3) ein nach außen führender Faden oder Doppelfaden (10) vorgesehen ist.

2. Verschluss nach Anspruch 1, wobei der Faden oder Doppelfaden (10) mehrere Drähte des Geflechtes umgreift.

3. Verschluss nach einem der Ansprüche 1 oder 2, wobei das Material der verschweißten Schirme oder Verschlussscheiben (3) und das diese bildende Geflecht aus Nitinoldraht besteht.

4. Verschluss nach einem der Ansprüche 1 bis 3, wobei die beiden beabstandeten Schirme oder Verschlussscheiben (3; 5) unterschiedliche Größen und/oder Formen haben.

5. Verschluss nach einem der Ansprüche 1 bis 4, wobei an jedem Schirm oder jeder Verschlussscheibe (3; 5) mehrere Drähte der Geflechte miteinander verdrillt und verdrillte Drähte der einen Verschlussscheibe mit solchen der anderen Verschlussscheibe verschweißt sind.

6. Verschluss nach einem der Ansprüche 1 bis 5, wobei die Schweißverbindung (4) mit aushärtendem Klebstoff und/oder flexiblem Kunststoff (11) ummantelt ist.

7. Verschluss nach Anspruch 6, wobei in dem Schirm oder die Verschlussscheibe (3) für wenigstens einen der Vorhöfe eine Kunststoffscheibe (7) integriert ist.

8. Verschluss nach Anspruch 7, wobei die Verschlussscheibe (3) oder der Schirm, der die Kunststoffscheibe (7) enthält, für deren Einsetzen bereichsweise offen ist.

## Claims

1. An occlusion device (100) for undesired openings in the heart (ASD, PFO, VSD) with two flexible welded umbrellas, spaced apart with respect to one another, serving as occlusion discs (3), made from a braid of metal wires, with which openings in the human heart are able to be closed and which are to be recovered and repositioned if necessary and in the case of incorrect positioning, wherein the connection between the umbrellas or occlusion discs (3) is free of clips, holders or threaded sleeves and is formed directly by a welded joint (4), and the metal wires of the braids forming the umbrellas or occlusion discs (3) are welded to one another on the sides facing one another of the umbrellas or occlusion discs (3), wherein a filament or double filament (10), leading outwards, is provided on the right-hand umbrella or on the occlusion disc (3) arranged on the right in the position of use.

2. The occlusion device according to claim 1, wherein the filament or double filament (10) encompasses several wires of the braid.

3. The occlusion device according to one of the claims 1 or 2, wherein the material of the welded umbrellas or occlusion discs (3) and the braid forming these consist of Nitinol wire.

4. The occlusion device according to one of the claims 1 to 3, wherein the two umbrellas or occlusion discs (3; 5), which are spaced apart, have different sizes and/or shapes.

5. The occlusion device according to one of the claims 1 to 4, wherein on each umbrella or on each occlusion disc (3; 5) several wires of the braids are twisted with one another, and twisted wires of one occlusion disc are welded to those of the other occlusion disc.

6. The occlusion device according to one of the claims 1 to 5, wherein the welded joint (4) is encased by hardenable adhesive and/or flexible adhesive (11).

7. The occlusion device according to claim 6, wherein a plastic disc (7) is integrated in the umbrella or the occlusion disc (3) for at least one of the atria.

8. The occlusion device according to claim 7, wherein the occlusion disc (3) or the umbrella, which contains the plastic disc (7), is open in some areas for the insertion thereof.

## Revendications

1. Fermeture (100) pour ouvertures indésirables dans le coeur (ASD, PFO, VSD) comprenant deux écrans en treillis de fils métalliques soudés, flexibles et espacés, servant de disques de fermeture (3), avec lesquels des ouvertures dans le coeur humain peuvent être fermées et qui, selon les besoins et les erreurs de mise en place, peuvent être retirés et placés de nouveau, sachant que la liaison entre les écrans ou disques de fermeture (3) est exempte d'agrafes, de douilles ou de manchons filetés et est formée directement par une soudure (4), et les fils métalliques des treillis formant les écrans ou les disques de fermeture (3) sont soudés entre eux sur les côtés des écrans ou des disques de fermeture (3) tournés l'un vers l'autre, sachant qu'un fil ou un double fil (10) guidant vers l'extérieur est prévu sur l'écran de droite en position d'utilisation ou sur les disques de fermeture (3) disposés à droite.

2. Fermeture selon la revendication 1, dans laquelle le fil ou le double fil (10) entoure plusieurs fils du treillis.

3. Fermeture selon la revendication 1 ou 2, dans laquelle le matériau des écrans ou disques de fermeture (3) soudés et le treillis formant ceux-ci sont en fil de nitinol.

4. Fermeture selon l'une des revendications 1 à 3, dans laquelle les deux écrans ou disques de fermeture (3 ; 5) espacés ont différentes tailles et/ou formes.

5. Fermeture selon l'une des revendications 1 à 4, dans laquelle plusieurs fils des treillis sont torsadés ensemble sur chaque écran ou disque de fermeture (3 ; 5) et des fils torsadés de l'un disque de fermeture sont soudés à des mêmes fils des autres disques de fermeture.

6. Fermeture selon l'une des revendications 1 à 5, dans laquelle la liaison soudée (4) est enveloppée d'adhésif durcissable et/ou d'adhésif flexible (11).

7. Fermeture selon la revendication 6, dans laquelle un disque en plastique (7) est intégré dans l'écran ou le disque de fermeture (3) pour au moins une des oreillettes.

8. Fermeture selon la revendication 7, dans laquelle le disque de fermeture (3) ou l'écran qui contient le disque en plastique (7) est ouvert par tronçons pour son introduction.
